# EUROPEAN PATENT APPLICATION

(11) **EP 0 897 928 A1**
(43) Date of publication of application: **24.02.1999**
(21) Application number: 98306525.1
(22) Date of filing: 17.08.1998
(51) Int. Cl.: C07K 1/00

(54) **Method for peptide synthesis**

(30) Priority: 19.08.1997 GB 9717431
(71) Applicant: University of Derby, Derby, DE22 1GB (GB)
(72) Inventor: Mellersh, Anthony Richard, Barrow-upon-Trent, Derby, DE73 1HQ (GB); Wilkinson, Alan Shaun, Derby, DE22 1GB (GB)
(74) Representative: Swindell & Pearson

(57) **Abstract**

A method of peptide synthesis comprising selecting an RNA to capture a required configuration of amino acids for a peptide in sequence. The RNA is attached onto a solid support before mixing with the amino acids which are then captured by the RNA. The mixture is then condensed to enable amide bonds to form between the amino acids thereby forming a peptide.

## Description

This invention relates to a method of peptide synthesis, and particularly but not exclusively polypeptide synthesis.

An increasing number of uses are being found for polypeptides and particularly in medicine, for example, synthetic peptides can serve as drugs and as antigens to stimulate the formation of specific antibodies. However, polypeptide synthesis is relatively complex and consequently expensive, and this expense often prevents the widespread use of such materials.

The term "capture" in this specification is to be understood as when a chemical compound physically receives another chemical compound to hold it, but not to bond with it.

According to the present invention there is provide a method of peptide synthesis, the method comprising selecting an RNA (Ribonucleic acid) which would capture a required configuration of amino acids for a peptide in sequence, attaching the RNA onto a solid phase, mixing the solid phase bearing the RNA with the required amino acids for the peptide such that the RNA captures the amino acids, subsequently condensing the mixture such that the amino acids separate from the RNA and form amides joined in the required configuration, with the RNA acting as a catalyst in the formation of the amides, and the structure of the peptide being dictated by the structure of the RNA.

The RNA preferably takes up a conformation of sequential clefts. Each cleft may be three bases long.

The clefts are preferably stabilised by the solid phase. Alternatively the clefts may be stabilised by chelation of cations by phosphates of adjacent clefts.

The mixture of solid phase material bearing RNA and amino acids, is preferably washed, for example with water, prior to condensation, to substantially remove all materials aside from the solid phase bearing the RNA, with the captured amino acids.

The amino acids are preferably in a dilute solution when mixed with the solid phase bearing the RNA, and the solution may be saline.

The clefts preferably capture, from the aqueous phase, the amino acid which the 5'-3' sequence in the cleft is that of the codon for the amino acid in the genetic code.

The RNA is preferably dried onto the solid phase material. The solid phase material may comprise silica which may be in the form of silica gel or sand. The silica gel is preferably acid washed silica gel. The RNA is preferably dried at 50°c

Organic solvents are preferably used in the condensation.

The amino acid solution is preferably incubated with the solid phase at a predetermined temperature for a predetermined period. The temperature is preferably 4°c. The period is preferably 1 hour. Preferably the captured amino acids are activated at the carboxylate by the RNA and this is preferably facilitated by changing the phase from aqueous to organic. Preferably deprotonation of the amino group of the initial amino acid leads to polymerisation of the amino acids into a peptide. Deprotonation preferably occurs when reacting the amino group with a base or alternatively to form an amide or other non positively charged derivature. Termination of the peptide synthesis may be achieved by an empty cleft or a termination codon.

The RNA is preferably recovered following the peptide synthesis and may be reused.

The invention also provides a method of polypeptide synthesis, the method being according to any of the preceding ten paragraphs.

Embodiments of the present invention will now be described by way of example only.

One embodiment relates to a laboratory procedure for showing that amide bonds can be produced from this invention, and thus it would be possible to synthesise peptides and particularly polypeptides by selecting the required RNA.

Poly A (100mg, Sigma Poole UK) is dissolved in distilled water (100ml) and dried onto silica gel (20g, Merck C60 230/70 mesh, 20mmHg/50°C). The resulting mixture (135mg) is placed in an Ependorf tube (1.50ml) and the amino acid solution added (1.00ml, 100mM). This is agitated (1 hour) in an ice bath to equilibrate.

Washing is then carried out on filter paper using a Buchner under reduced pressure at room temperature. Washing and drying then takes place with methanol.

The resulting compound has ammonia added in excess (typically 50 millimoles) in 20ml of toluene. This was then dried and purified. Analysis is then carried out to illustrate that amide bonds had been formed, being bonds between lysine and ammonia. This analysis was carried out using GC mass spectrometry and HPLC. This system can be extended to peptide synthesis.

As noted above, to produce particular peptides and particularly polypeptides, a different and more complex RNA than Poly A would be selected. Such an RNA would have a structure to capture the required amino acids in the required configuration for the peptide. The RNA can take up a conformation of sequential clefts each three bases long. The clefts are stabilised by either a solid phase (such as silica gel) or the chelation of cations by the phosphates of adjacent clefts. These clefts will capture (from aqueous phase) the amino acid which the 5'-3' sequence of the cleft is that of the codon for the amino acid in the genetic code (attached). Obviously appropriate amino acids would be added to the RNA when attached to a solid phase material. The captured amino acids are activated at the carboxylate by the RNA, a process facilitated by changing the phase from aqueous to organic. Deprotonation of the amino group of the initial amino acid leads to polymerisation of the amino acid into a peptide (or larger peptide, polypeptide or protein). Termination is achieved by an empty cleft or a termination codon.

To illustrate making a specific peptide, say NH2-Met-Lys-Leu-Ser-COOH, RNA containing or consisting of sequence 5'-AUGAAAUU-GUCG-3' is attached to a solid phase, typically acid washed silica gel. This would easily be accomplished by drying it on at 50°c.

An aqueous solution containing amino acids, especially Methionine, Lysine, Leucine and Serine, is then added before incubating at 4°c for a period such as 1 hour. The phase is then changed from an aqueous to an organic phase.

The Methionine is then either deprotonated at the NH₃+ with a base or it is reacted to form an amide or other non positively charged derivative.

When the peptide has polymerised, the final mixed anhydride of the carboxylate of serine and the phosphate is hydrdysed to release the peptide.

The process is repeated by returning to the step where the solution of amino acids is added.

There is thus described a method of peptide synthesis which provides for extremely consistent results. Once the appropriate RNA had been selected, this will accurately accept the appropriate amino acid in the appropriate configuration. The RNA is stereospecific with regard to capture sides. The method can be relatively inexpensively and consistently performed once in production thereby producing, for instance, polypeptides at a greatly reduced cost relative to conventional techniques. The peptides can be easily separated from the RNA on the solid phase, and the RNA is recovered after peptide synthesis and can be reused.

As noted above the particular RNA and amino acids for each peptide will be used. Peptides of any length or amino acid sequence could be made by altering the RNA length and sequence. Also, different conditions and other constituents can be chosen to suit particular materials.

Whilst endeavouring in the foregoing specification to draw attention to those features of the invention believed to be of particular importance it should be understood that the Applicant claims protection in respect of any patentable feature or combination of features hereinbefore referred to and/or shown in the drawings whether or not particular emphasis has been placed thereon.

## Claims

1. A method of peptide synthesis, the method comprising selecting an RNA (Ribonucleic acid) which would capture a required configuration of amino acids for the peptide to be synthesised, attaching the RNA onto a solid phase, mixing the solid phase bearing the RNA with the required amino acids for the peptide such that the RNA captures the amino acids, subsequently condensing the mixture such that the amino acids separate from the RNA and form amides joined in the required configuration, with the RNA acting as a catalyst in the formation of the amides, and the structure of the peptide being dictated by the structure of the RNA.

2. A method according to claim 1 characterised in that the RNA takes up a conformation of sequential clefts.

3. A method according to claim 2 characterised in that each cleft is three bases long.

4. A method according to claims 2 or 3 characterised in that the clefts are stabilised by the solid phase.

5. A method according to claims 2 or 3 characterised in that the clefts are stabilised by chelation of cations by phosphates of adjacent clefts.

6. A method according to any of the preceding claims characterised in that the mixture of solid phase material bearing RNA and amino acids, is washed, prior to condensation, to substantially remove all materials aside from the solid phase bearing the RNA, with the captured amino acids.

7. A method according to claim 6 characterised in that the mixture of solid phase material bearing RNA and amino acids is washed with water.

8. A method according to any of the preceding claims characterised in that the amino acids are in a dilute solution when mixed with the solid phase bearing the RNA,

9. A method according to claim 8 characterised in that the solution is saline.

10. A method according to any of claims 2 to 9 characterised in that the clefts capture, from the aqueous phase, the amino acid which the 5'-3' sequence in the cleft is that of the codon for the amino acid in the genetic code.

11. A method according to any of the preceding claims characterised in that the RNA is dried onto the solid phase material.

12. A method according to any of the preceding claims characterised in that the solid phase material comprises silica

13. A method according to claim 12 characterised in that the silica is in the form of silica gel.

14. A method according to claim 12 characterised in that the silica is in the form of sand.

15. A method according to any of claims 12 to 14 characterised in that the silica gel is acid washed silica gel.

16. A method according to any of claims 11 to 15 characterised in that the RNA is dried at 50°c

17. A method according to any of the preceding claims characterised in that organic solvents are used in the condensation.

18. A method according to any of the preceding claims characterised in that the amino acid solution is incubated with the solid phase at a predetermined temperature for a predetermined period.

19. A method according to claim 18 characterised in that the temperature is 4°c.

20. A method according to claim 18 or 19 characterised in that the period is 1 hour.

21. A method according to any of the preceding claims characterised in that the captured amino acids are activated at the carboxylate by the RNA.

22. A method according to claim 21 characterised in that the activation is preferably facilitated by changing the phase from aqueous to organic.

23. A method according to any of the preceding claims characterised in that deprotonation of the amino group of the initial amino acid leads to polymerisation of the amino acids into a peptide.

24. A method according to claim 23 characterised in that deprotonation occurs when reacting the amino group with a base or alternatively to form an amide or other non positively charged derivature.

25. A method according to any of the preceding claims characterised in that termination of the peptide synthesis is achieved by an empty cleft or a termination codon.

26. A method according to any of the preceding claims characterised in that the RNA is recovered following the peptide synthesis and can be reused.

27. A method of polypeptide synthesis, the method being according to any of the preceding claims.
